# EUROPEAN PATENT APPLICATION

(11) **EP 4 840 871 A1**
(43) Date of publication of application: **18.11.2026**
(21) Application number: 25869576.6
(22) Date of filing: 27.06.2025
(51) Int. Cl.: C07J 9/00, A61K 31/575, A61P 35/00

(54) **COMPOUND HAVING ANTI-TUMOR ACTIVITY, AND PREPARATION METHOD THEREFOR AND USE THEREOF**

(30) Priority: 25.06.2025 CN 202510856629
(71) Applicant: SICHUAN LANYING PHARMACEUTICAL CO., LTD., Leshan, Sichuan 614200 (CN)
(72) Inventor: The designation of the inventor has not yet been filed
(74) Representative: Patrade A/S
(86) International application number: PCT/CN2025/104447
(87) International publication number: WO 2026/148804

(57) **Abstract**

Provided is an anti-tumor compound having the chemical structure shown in formula (I). This compound is a novel pharmaceutical natural product of tetracyclic triterpenoids. It exhibits favorable lipophilicity and chemical stability, can be conveniently extracted from the rhizomes of the herbal plant *Begonia limprichtii Irmscher,* and can be formulated into oral or injectable dosage forms. Further provided is a method for preparing said compound and use thereof. The compound of the present invention can significantly inhibit the growth of mouse lung cancer, reduce tumor volume and weight, and is useful for tumor treatment.

## Description

### TECHNICAL FIELD

The present invention relates to the field of medicine, and specifically to a novel pharmaceutical natural product of tetracyclic triterpenoids. More specifically, it relates to an anti-tumor compound, a preparation method, and use thereof.

### BACKGROUND

Tumors pose a serious threat to human life and health with their incidence and mortality rates continuing to rise worldwide. Current methods for treating tumors mainly include surgery, radiotherapy, chemotherapy, and immunotherapy. Although significant progress has been made in anti-tumor biotechnology in recent years, chemotherapy remains one of the most commonly used fundamental approaches in clinical practice. Its applicable candidates include: systemic malignant tumors sensitive to chemotherapy, such as leukemia, multiple myeloma, and intermediate-to-high grade lymphomas; advanced tumors with no indications for surgery or radiotherapy, or patients with recurrence and metastasis after surgery or radiotherapy; solid tumors requiring preoperative neoadjuvant or postoperative adjuvant therapy; and as an adjunct prior to radiotherapy.

Chemotherapy primarily utilizes small-molecule chemical drugs derived from synthesis or plant extraction, including alkaloids, platinum-based compounds, targeted kinase inhibitors, aromatase inhibitors, alkylating agents, taxanes, etc. There are numerous types of drugs, ranging from cell cycle non-specific agents (CCNSA) that kill cells throughout the entire proliferation cycle to cell cycle specific agents (CCSA) that only kill cells during a specific phase. Combination therapy often yields better efficacy. Due to its broad applicability and low cost, chemotherapy will undoubtedly remain a major means of anti-tumor treatment in the future.

However, although existing chemotherapeutic drugs are recognized for their tumor-inhibiting effects and remain a common and effective treatment approach, they suffer from numerous drawbacks, including extensive and severe toxic side effects, as well as the development of drug resistance. Due to their poor selectivity between tumor cells and normal cells, the drugs damage health cells while inhibiting or killing tumor cells, which impairs cardiac, hepatic, renal, and neurological functions, leading to systemic toxicity in the human body.

To address these challenges, many researchers aim at exploring novel anti-tumor structures, with a particular focus on identifying bioactive constituents from the plant kingdom for anti-tumor drug development. For instance, piperine, known for its various pharmacological activities, entered Phase II clinical trials in the United States in 2021 for the treatment of multiple myeloma and prostate cancer. Another example is steroidal saponins derived from Paris polyphylla var. *yunnanensis*, which serves as the primary active constituent found in such plant, has demonstrated a certain inhibitory effect on pancreatic cancer cells; however, their therapeutic effects need improvement. Consequently, there is an urgent need to discover a novel anti-tumor compound with low toxicity, high efficiency, strong specificity, and favorable pharmacokinetic properties.

*Begonia limprichtii Irmscher* (commonly known as "Daoshuilian" in Chinese) is a folk medicinal herb indigenous to southwestern China. As a perennial herbaceous species of the family Begoniaceae, it has been employed to treat injuries from falls and blows, rheumatic pain, sore throat, etc. This herb exhibits favorable safety with no significant toxic side effects. However, to date, there have been no reports on its chemical constituents and anti-tumor activities of this medicinal material.

### SUMMARY

To address the above-mentioned technical problems, it is an object of the present invention to provide a small-molecule compound with a novel structure and potent anti-tumor activity, serving as a novel therapeutic alternative drug for the treatment of the tumors.

To achieve the above object, the present invention provides the following technical solutions:
In a first aspect, the present invention provides an anti-tumor compound having the chemical structural formula as shown in Formula (I):

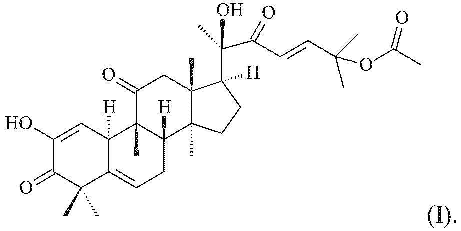

The aforementioned compound, named "Daoshuilianin A", features a parent nucleus containing 30 carbon atoms, making it a typical tetracyclic triterpenoid skeleton. Its structural feature is characterized by the attachment of the angular methyl group 19-CH₃ to C-9, rather than the conventional C-10 position in triterpenoids. While it also belongs to the cucurbitacin class, it lacks the hydroxyl substitution at C-16 commonly found in cucurbitacins, thus representing a rare 16-dehydroxycucurbitacin. This novel structure has not been reported previously. Daoshuilianin A is a white powder with a bitter taste, sparingly soluble in water, freely soluble in organic solvents such as methanol, ethanol, ethyl acetate, and chloroform. Stability studies demonstrated that no reduction in its content and no alterations in properties are observed after 12 months of storage at room temperature. Under accelerated testing conditions of 40°C and 75% relative humidity for 6 months, no significant alterations in various physicochemical properties were observed either. These indicate that the compound possesses high chemical stability, providing favorable conditions for the manufacture, transport, and storage of its pharmaceutical preparations.

In a second aspect, it is another object of the present invention to provide a pharmaceutical composition comprising the compound of the present invention.

Preferably, said pharmaceutical composition further comprises a pharmaceutically acceptable carrier and/or excipient.

In a third aspect, it is another object of the present invention to provide a method for preparing the compound of the present invention, comprising the following steps:
a. Providing dried rhizomes of *Begonia limprichtii Irmscher,* pulverizing them, extracting with an organic solvent, filtering, and concentrating to obtain a fluid extract;
b. Adding the fluid extract to water, stirring, filtering, washing with water, and drying to obtain a precipitate;
c. Separating the precipitate by silica gel column chromatography, eluting with a gradient of petroleum ether-ethyl acetate; and
d. Monitoring the fractions by silica gel column chromatography, combining, and concentrating to obtain the compound.

Preferably, the organic solvent in step (a) may be any one selected from methanol, ethanol, ethyl acetate, acetone, chloroform, petroleum ether, or a mixture thereof. Exemplary mixture include such as a combination of petroleum ether and ethyl acetate (e.g., in a volume ratio of 2:1). More preferably, the organic solvent is lower alcohol, such as methanol or ethanol. and even more preferably, pharmaceutical alcohol, i.e., 75% to 85% (v/v) aqueous ethanol, to comply with relevant pharmaceutical production regulations, cost-effectiveness, safety, and environmental friendliness. The extraction method may be performed by percolation, reflux, maceration, microwave, or supercritical extraction, preferably by reflux.

Preferably, the weight-to-volume ratio of *Begonia limprichtii Irmscher* to the organic solvent is 1 g : 3 to 5 mL, more preferably 1 g : 4 mL.

Preferably, step (b) is a water precipitation process, wherein water is added in an amount of 5 to 15 times the weight of the extract, preferably 10 times. The mixture is stirred at room temperature or under heating to fully dissolve and suspend the paste. Subsequently, the mixture is cooled, allowed to stand, and then filtered. The precipitate is washed repeatedly with small portions of water.

Preferably, the column chromatography in step (c) may be repeated until the target compound with the desired purity is obtained.

Preferably, in said step (d), the column chromatography fractions are monitored by thin-layer chromatography (TLC) or high-performance liquid chromatography (HPLC).

More preferably, said thin-layer chromatography is performed on conventional silica gel plates, using petroleum ether-ethyl acetate (3:1) or chloroform-methanol (98:2) as the developing solvent system, with visualization by iodine vapor staining method.

More preferably, the liquid chromatography method uses a conventional C18 reversed-phase silica gel column, with a mobile phase of methanol-0.1% formic acid (50:50 to 100:0) under gradient elution, and a detection wavelength of 237 nm.

The preparation method of the present invention features a short extraction process, simple operation, and low cost, with column chromatography packing being reusable, thereby making it feasible for scaled-up production.

In a fourth aspect, it is an object of the present invention to provide use of the compound or pharmaceutical composition of the present invention in the preparation of an anti-tumor medicament.

Preferably, the tumor includes, but is not limited to, lung cancer, breast cancer, liver cancer, colon cancer, rectal cancer, and gastric cancer. More preferably, the lung cancer is squamous cell lung carcinoma and non-small cell lung cancer (NSCLC).

Anti-tumor assays demonstrate that the compound of the present invention exhibits highly significant inhibitory activity *in vitro* against human-derived tumor cell lines, including lung cancer, liver cancer, gastric cancer, breast cancer, and colon cancer, with IC₅₀ values ranging from 0.8251 to 38.63 µg/mL. Further, *in vivo* animal studies show that oral administration by gavage at a dose of 40 mg·kg⁻¹·d⁻¹ for 9 days can significantly inhibit the tumor growth in LLC tumor-bearing mice, reducing tumor volume and weight. Thus, Daoshuilianin A has significant anti-tumor effects in both *in vitro* and *in vivo* models, with an anti-tumor spectrum covering five common solid tumors: lung cancer, breast cancer, liver cancer, gastric cancer, and colon cancer, exhibiting broad-spectrum activity, and potential inhibitory effects on additional tumor types cannot be excluded.

Preferably, the compound is formulated into a pharmaceutically acceptable dosage form either as a pure compound or as a plant extract containing such compound.

Preferably, the dosage form comprises a pharmaceutically acceptable carrier and/or excipient.

Pure Daoshuilianin A or plant extracts containing Daoshuilianin A can be prepared into various pharmaceutically acceptable dosage forms to meet the diverse requirements of clinical medication. These dosage forms include, but are not limited to, tablets, hard capsules, soft capsules, granules, dropping pills, ointments, and injections. The pharmaceutically acceptable carriers, excipients, or additives required for preparations specifically include, for example, pharmaceutically commonly used diluents and absorbents such as starch, dextrin, calcium sulfate, lactose, mannitol, sucrose, sodium chloride, glucose, calcium carbonate, kaolin, microcrystalline cellulose, aluminum silicate, calcium hydrogen phosphate, light magnesium oxide, etc.; pharmaceutically commonly used wetting agents and binders such as water, glycerin, polyethylene glycol, ethanol, propanol, starch paste, dextrin, syrup, honey, glucose solution, gum arabic mucilage, gelatin mucilage, sodium carboxymethyl cellulose, shellac, methyl cellulose, potassium phosphate, polyvinylpyrrolidone, etc.; pharmaceutically commonly used disintegrants such as dry starch, alginates, agar powder, laminarin starch, sodium bicarbonate, citric acid, calcium carbonate, polyoxyethylene, sorbitan fatty acid esters, sodium dodecyl sulfonate, methyl cellulose, ethyl cellulose, etc.; disintegration inhibitors such as sucrose, glyceryl tristearate, cocoa butter, hydrogenated oils, etc.; lubricants such as talc, silicon dioxide, corn starch, stearates, boric acid, liquid paraffin, polyethylene glycol, etc.; pharmaceutically commonly used co-solvents such as ethanol, propylene glycol, polyethylene glycol, polysorbate 80, lecithin, urea, sodium benzoate, sodium salicylate, nicotinamide, cyclodextrin, soybean oil, etc. Pharmaceutically acceptable carriers or excipients are not exhaustively listed herein, and one skilled in the art can select specifical ones based on common general knowledge.

Compared with the prior art, the present invention has the following beneficial effects:
(1) Daoshuilianin A is a novel small-molecule component with a structural type entirely distinct from existing drugs, suggesting the potential for different therapeutic targets and mechanisms of action;
(2) Daoshuilianin A is highly efficient and broad-spectrum in anti-tumor activity, with an estimated single daily oral dose for adults of less than 100 mg;
(3) The source plant has a long history of use as a folk medical herb with no obvious toxic side effects, indicating that Daoshuilianin A possesses good clinical safety;
(4) The source plant is a herbaceous species of the genus *Begonia* Linn., similar to common horticultural begonia flower varieties. It can be artificially propagated and cultivated on a large scale, thereby meeting the demand for raw materials in mass production;
(5) The preparation process of Daoshuilianin A is simple, enabling to obtain high-purity compounds at low cost;
(6) Daoshuilianin A has strong lipophilicity and good solubility, facilitating easy oral absorption;
(7) Daoshuilianin A exhibits stable physicochemical properties and has a long shelf life.

In summary, the Daoshuilianin A of the present invention has a novel structure and outstanding advantages such as high efficiency, a novel mechanism, and high stability. With readily available raw materials and low cost, it represents a novel anti-tumor drug with great development potential.

### BRIEF DESCRIPTION OF THE DRAWINGS

Figure 1 depicts the mass spectrum of Daoshuilianin A.
Figure 2 depicts the ¹H-NMR spectrum of Daoshuilianin A.
Figure 3 depicts the ¹³C-NMR spectrum of Daoshuilianin A.
Figure 4 depicts the HPLC chromatogram of Daoshuilianin A.
Figure 5 depicts the ultraviolet spectrum of Daoshuilianin A.
Figure 6 depicts the dose-response curves of Daoshuilianin A inhibiting the proliferation of 13 tumor cell lines.
Figure 7 shows the inhibitory effect of Daoshuilianin A on tumor growth in lung cancer-bearing mice. (A) Tumor growth profiles in mice; (B) Comparison of tumor size; (C) Comparison of tumor volume; (D) Comparison of tumor weight.

### DETAILED DESCRIPTION OF THE INVENTION

The technical solutions of the present invention are further described below with reference to the accompanying drawings and specific embodiments. Unless otherwise specified, the instruments or reagents used in the examples are conventional instruments or reagents in the art, which are commercially available conventional products. Unless otherwise specified, the specific experimental operations involved herein are understandable or known to those of one skilled in the art based on common general knowledge or conventional technical means, and thus will not be redundantly described herein.

### Embodiment 1: Extraction, Isolation, and Structure Characterization of Daoshuilianin A

### 1. Example 1: Extraction and Isolation of Daoshuilianin A

(1) 300 g of dried rhizomes of *Begonia limprichtii Irmsch*. (known in Chinese as "Daoshuilian" or "Jiyeqiuhaitang"), purchased from a herbal market in Leshan City, Sichuan Province were crushed into coarse powder. The powder was placed in a round-bottom flask, extracted with 1.2 L of methanol under reflux for 1 hour, cooled, and filtered to obtain an extracting solution. The residue was subjected to the same extraction procedure two additional times and another two extracting solutions were obtained. The three extracting solutions were combined and concentrated under reduced pressure to yield 65 g of extract.
(2) The extract was poured into 650 mL of water, heated in a water bath at 80°C, and stirred rapidly to ensure full dissolution, dispersion, and suspension of the extract. The mixture was then cooled, allowed to stand for 1 hour, and filtered. The filter cake was washed sequentially with small portions of water and dried to obtain 33 g of precipitated powder.
(3) 20 g of the precipitated powder was dissolved in 60 mL of methanol, mixed with silica gel, and dried. The mixture was loaded onto a silica gel column (silica gel specification: 200-300 mesh) and eluted with a gradient of petroleum ether-ethyl acetate (10:0 to 0:10). A total of 80 fractions (Fr. 1 to Fr. 80) were collected. Each fraction was analyzed by silica gel thin-layer chromatography (TLC) using petroleum ether-ethyl acetate (3:1, v/v) as the developing system and visualized by iodine vapor staining. The results showed that Fractions 15 to 18 (Fr. 15 to Fr. 18) contained the same single major spot. These fractions were combined and concentrated to yield 1.8 g of white powder (designated as PSY-2).

The purity of PSY-2 was determined by high performance liquid chromatography (HPLC) under the following conditions: Column: ODS column (150 × 4.6 mm, 5 µm); Mobile Phase: Methanol and 0.1% formic acid, gradient elution from 50:50 to 100:0; Detection Wavelength: 237 nm; Flow Rate: 1.0 mL/min.

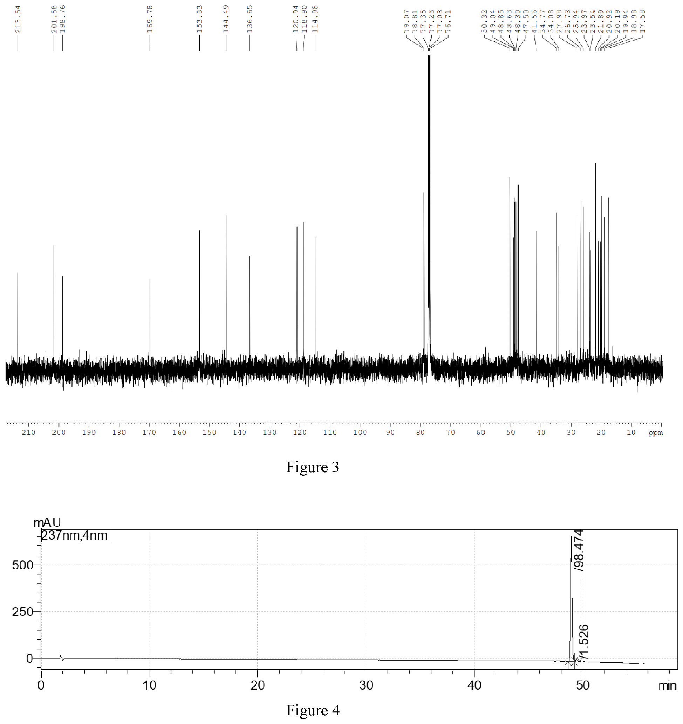

As shown in Figure 4, the chromatogram displayed a single major peak with a purity of >98%.

### 2. Structure Characterization

Pure Daoshuilianin A appeared as a white powder with a bitter taste, and was insoluble in water but readily soluble in organic solvents such as methanol, ethanol, ethyl acetate, and chloroform. Stability studies indicated that the content of the compound remained unchanged, and no alterations in physical properties were observed after storage at room temperature for 12 months. Furthermore, under accelerated testing conditions (40°C, 75% relative humidity) for 6 months, no significant changes in any physicochemical indicators were detected, demonstrating its chemical stability.

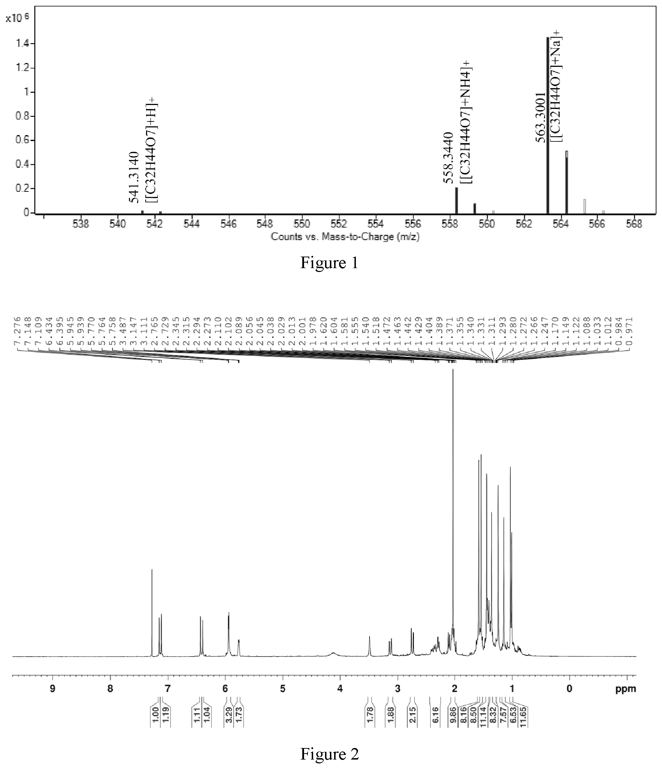

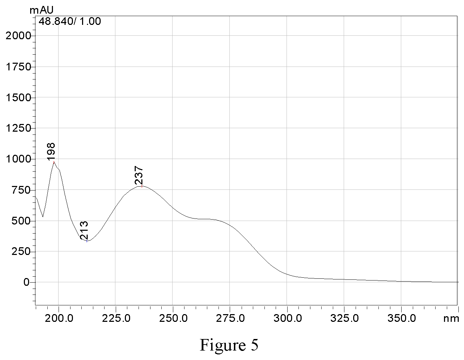

[α]20,D +11.82° (c0.11, MeOH), UV λmax : 237, 266(sh)nm (Figure 5); ESI-TOF-MS m/z: 541.3140 [M+H]⁺, 558.3440 [M+NH₄]⁺, 563.3001 [M+Na]⁺ (Calculated for C₃₂H₄₄O₇, 540.308704)(Figure 1).

¹H-NMR(400MHz, CDCl₃) δppm: 1.03 (3H, s, H-18), 1.09 (3H, s, H-19), 1.15 (3H, s, H-29), 1.25 (3H, s, H-28), 1.36 (3H, s, H-30), 1.44 (3H, s, H-21), 1.54 (3H, s, H-26), 1.58 (3H, s, H-27), 2.03 (3H, s, Ac), 5.76 (1H, m, H-6), 5.94 (1H, d, J=2.4Hz, H-1), 6.41 (1H, d, J=15.6Hz, H-23), 7.12 (1H, d, J=15.6Hz, H-24), 3.49 (1H, br s, H-10), 3.13 (1H, d, J=14.4Hz, H12b), 2.74 (1H, d, J=14.4Hz, H12a), 2.33 (1H, m, H-7a), 2.03 (1H, m, H-7b), 2.04 (1H, m, H-8), 0.9~1.7 (5H, m, H-15,16,17) (Figure 2).

¹³C-NMR(100MHz, CDCl₃) δppm: 114.98 (C-1), 144.49 (C-2), 198.76 (C-3), 47.50 (C-4), 136.65 (C-5), 120.94 (C-6), 23.54 (C-7), 41.56 (C-8), 48.85 (C-9), 34.77(C-10), 213.54(C-11), 48.63(C-12), 50.32(C-13), 48.30(C-14), 34.08(C-15), 20.92(C-16), 49.04(C-17), 18.98(C-18), 19.94(C-19), 78.81(C-20), 23.97(C-21), 201.58(C-22), 118.90(C-23), 153.33(C-24), 79.07(C-25), 26.73(C-26), 25.94(C-27), 27.98(C-28), 20.19(C-29), 17.58(C-30), 21.89(Ac), 169.78(Ac) (Figure 3).

Based on the analysis of 2D spectra including HSQC and HMBC and with reference to relevant literature (Magn. Reson. Chem. 2005, 43, 489-491), the above spectral data were fully assigned. PSY-2 was identified as a novel cucurbitacin and named Daoshuilianin A.

The molecular formula of Daoshuilianin A (designated as PSY-2) is C₃₂H₄₄O₇, with a molecular weight of 540. Its molecular structure is shown in Formula (I) below.

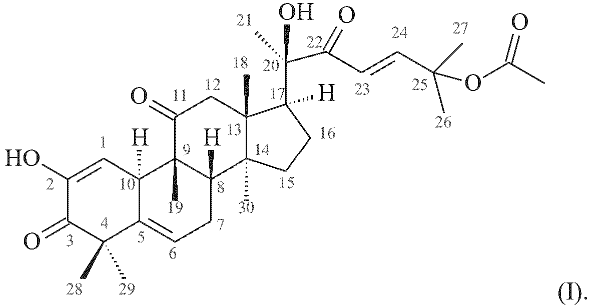

The systematic name of Daoshuilianin A is (6R,E)-6-hydroxy-6-((9R,10R,13R,14S,17S)-2-hydroxy-4,4,9,13,14-pentamethyl-3,11-dioxo-4,7,8,9,10,11,12,13,14,15,16,17-dodecahydro-3H-cyclopenta[a]phenanthren-17-yl)-2-methyl-5-oxohept-3-en-2-yl acetate.

### 3. Example 2: Extraction and Isolation of Daoshuilianin A

(1) 100 g of dried *Begonia limprichtii Irmscher* (Daoshuilian) was crushed into coarse powder and placed in a round-bottom flask. 500 mL of 80% (v/v) ethanol was added, and the mixture was extracted under reflux for 1 hour. After cooling, the mixture was filtered to obtain an extracting solution. The residue was subjected to the same extraction procedure two additional times and another two extracting solutions were obtained. The three extracting solutions were combined and concentrated under reduced pressure to yield 22 g of extract.
(2) The extract was poured into 330 mL of water, heated in a water bath at 80°C, and stirred rapidly to ensure full dissolution, dispersion, and suspension of the extract. The mixture was then cooled, allowed to stand for 1 hour, and filtered. The filter cake was washed sequentially with small portions of water and dried to obtain 13 g of precipitated powder.
(3) 10 g of the precipitated powder was dissolved in 30 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Each fraction was analyzed by silica gel thin-layer chromatography (TLC) using petroleum ether-ethyl acetate (3:1, v/v) as the developing system and visualized by iodine vapor staining. Based on the TLC results, appropriate fractions were combined and concentrated to yield 1.2 g of pale yellow powder.
(4) The obtained powder was dissolved in 5 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Guided by TLC analysis, the fractions were combined and concentrated to yield 0.7 g of white powder.

### 4. Example 3: Extraction and Isolation of Daoshuilianin A

(1) 100 g of dried *Begonia limprichtii Irmscher* was crushed into coarse powder and placed in a round-bottom flask. 300 mL of ethyl acetate was added, and the mixture was extracted under reflux for 1 hour. After cooling, the mixture was filtered to obtain an extracting solution. The residue was subjected to the same extraction procedure two additional times and another two extracting solutions were obtained. The three extracting solutions were combined and concentrated under reduced pressure to yield 13 g of extract.
(2) The extract was poured into 65 mL of water, heated in a water bath at 80°C, and stirred rapidly to ensure full dissolution, dispersion, and suspension of the extract. The mixture was then cooled, allowed to stand for 1 hour, and filtered. The filter cake was washed sequentially with small portions of water and dried to obtain 11 g of precipitated powder.
(3) 10 g of the precipitated powder was dissolved in 30 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Each fraction was analyzed by silica gel TLC using petroleum ether-ethyl acetate (3:1, v/v) as the developing system and visualized by iodine vapor staining. The fractions were combined and concentrated to yield 0.9 g of white powder.

### 5. Example 4: Extraction and Isolation of Daoshuilianin A

(1) 100 g of dried *Begonia limprichtii Irmscher* was crushed into coarse powder and placed in a round-bottom flask. 350 mL of petroleum ether-ethyl acetate (2:1, v/v) was added, and the mixture was extracted under reflux for 1 hour. After cooling, the mixture was filtered to obtain an extracting solution. The residue was subjected to the same extraction procedure two additional times and another two extracting solutions were obtained. The three extracting solutions were combined and concentrated under reduced pressure to yield 12 g of extract.
(2) The extract was poured into 90 mL of water, heated in a water bath at 80°C, and stirred rapidly to fully dissolve, disperse, and suspend the extract. The mixture was then cooled, allowed to stand for 1 hour, and filtered. The filter cake was washed sequentially with small portions of water and dried to obtain 11 g of precipitated powder.
(3) 10 g of the precipitated powder was dissolved in 30 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Each fraction was analyzed by silica gel TLC using petroleum ether-ethyl acetate (3:1, v/v) as the developing system and visualized by iodine vapor staining. The fractions were combined and concentrated to yield 1.0 g of white powder.

Chemical structure characterization confirmed that the products obtained from the extraction and isolation describes in Examples 2 to 4 above were identical to Daoshuilianin A (PSY-2).

### Embodiment 2: Inhibitory Effect of Daoshuilianin A (PSY-2) on Tumor Cells

### 1. Reagents, Materials, and Culture conditions

All cell lines were commercially available and were cultured under the following conditions:
NCI-H226 (Human non-small cell lung cancer, squamous cell carcinoma; ATCC CRL-5826): RPMI-1640 medium + 10% fetal bovine serum (FBS) + 1% penicillin-streptomycin (PS).
HCC827 (Human non-small cell lung cancer; ATCC CRL-2868): RPMI-1640 medium + 10% FBS + 1% PS.
HepG2 (Human hepatocellular carcinoma; ATCC HB-8065): MEM (with NEAA) medium + 10% FBS + 1% PS.
Hep3B (Human hepatocellular carcinoma; ATCC HB-8064): MEM (with NEAA) medium + 10% FBS + 1% PS.
HCT116 (Human colorectal carcinoma; ATCC CCL-247): McCoy's 5A medium + 10% FBS + 1% PS.
HT29 (Human colorectal carcinoma; ATCC HTB-38): McCoy's 5A medium + 10% FBS + 1% PS.
MKN-45 (Human poorly differentiated gastric adenocarcinoma; Wuhan Pricella Biotechnology Co., Ltd., CL-0292): RPMI-1640 medium + 10% FBS + 1% PS.
BGC-823 (Human gastric adenocarcinoma; Shanghai GuanDao Bio Engineering Co., Ltd., C1953): RPMI-1640 medium + 10% FBS + 1% PS.
MCF-7 (Human breast adenocarcinoma; ATCC CRL-3435): DMEM medium + 10% FBS + 1% PS.
MDA-MB-231 (Human breast adenocarcinoma; ATCC CRM-HTB-26): DMEM medium + 10% FBS + 1% PS.
H1975 (Human non-small cell lung cancer; ATCC CRL-5908): F12/DMEM medium + 10% FBS + 1% PS.
A549 (Human lung adenocarcinoma epithelial cells; ATCC CRM-CCL-185): F12/DMEM medium + 10% FBS + 1% PS.
H1299 (Human non-small cell lung cancer; ATCC CRL-5803): F12/DMEM medium + 10% FBS + 1% PS.

Reagents: Cell culture media (GIBCO); fetal bovine serum (GIBCO); trypsin-EDTA (GIBCO); CCK-8 Kit (Meilun Biotechnology, MA0218); penicillin-streptomycin (PS, Sigma-Aldrich).

Instruments: CO₂ Incubator (Thermo Fisher Scientific); Multiskan Spectrum (BioTek, Epoch); High-speed Benchtop Centrifuge (Eppendorf, 5804R); Laminar Flow Cabinet (Shanghai Lu Jing Clean Equipment, SW-CJ-2D); Inverted Microscope (Olympus).

### 2. Experimental Methods

### 2.1 Cell Resuscitation

(1) Cryovials were removed from liquid nitrogen tank and immediately placed in a 37°C water bath. The vials were gently agitated to facilitate rapid thawing.
(2) Upon removal from the water bath, the cryovials were disinfected with 75% (v/v) ethanol and wiped dry. The caps were opened, and the cell suspension was aspirated using a pipette into a centrifuge tube. More than 5 volumes of complete culture medium were added dropwise and mixed gently.
(3) The mixture was centrifuged at 1,000 rpm for 5 minutes.
(4) The supernatant was discarded, and the cell pellet at the bottom of the tube was resuspended. The cells were seeded into a T25 culture flask containing 5 to 6 mL of complete medium. Cell counting was performed to adjust the cell density before incubation. The flasks were incubated statically at 37°C.
(5) The medium was replaced with fresh complete culture medium the next day, and cultivation was continued.

### 2.2 Cell Subculturing (Passaging)

(1) Cells were subcultured upon reaching 90% confluence in the culture flask.
(2) For a T25 culture flask, the complete medium was aspirated, and the cells were gently rinsed once with PBS buffer. Then, 0.5 mL of 0.25% trypsin-EDTA was added, and the cells were digested for 2 minutes (the digestion time may be adjusted appropriately depending on the cell type). Subsequently, 1 mL of complete medium was used to rinse the cells off the surface. The suspension was centrifuged at 1,000 rpm for 5 minutes. The supernatant was removed, and the collected cells were seeded into two new T25 culture flasks. 5 mL of complete medium was added to each flask for further cultivation.

### 2.3 Cell Counting Analysis

(1) The digested cells were thoroughly resuspended and pipetted repeatedly to disperse them into a single-cell suspension. 1 mL of the suspension was transferred into a clean EP tube.
(2) The surface of the hemocytometer (cell chamber) and the coverslip were gently wiped with alcohol-soaked cotton balls. The edges of the coverslip were slightly moistened with the cotton balls, and it was carefully placed over the center of the cell chamber, ensuring that the edges covered the grids on the hemocytometer.
(3) The cell suspension previously transferred to the EP tube was resuspended again by repeated pipetting.
(4) 20 µL of the aforementioned cell suspension was aspirated. The pipette tip was positioned against the upper or lower edge of the coverslip, and the liquid was dispensed slowly. Due to siphonage action, the liquid was drawn into the space between the coverslip and the counting chamber.
(5) The microscope was adjusted, and observation was performed using a 10× objective lens.
(6) According to standard cell counting procedures, viable cells (those excluding trypan blue) were counted.
(7) After counting, the cell concentration was calculated using the following formula:$Cell Concentration \left(cells/mL\right) = \left(Total number of cells in 4 large squares / 4\right) \times 10,000 .$
(8) The total number of cells was further calculated as follows:$Total Cell Number = Volume of resuspended culture medium \left(mL\right) \times Cell Density \left(cells/mL\right) .$

### 2.4 Cell Viability Assay (CCK-8)

### Experimental Procedure:

(1) Cells were digested and counted. The cell concentration was adjusted to 5 × 10⁴ cells/mL, and 100 µL of the cell suspension was seeded into each well of a 96-well plate.
(2) After overnight incubation in the plate, the culture medium was removed, and 100 µL of fresh culture medium containing the test compound at various concentrations was added to each well. Following 24-hours treatment, the drug-containing medium was discarded, and CCK-8 working solution was added, wherein the working solution was prepared by mixing CCK-8 reagent with culture medium at 1:10 volume ratio.
(3) The plate was incubated in the cell culture incubator for 3 hours, and the absorbance of the CCK-8 working solution was measured at 450 nm.

### 2.5 Calculation of Half-Maximal Inhibitory Concentration (IC₅₀)

IC₅₀ values were calculated using GraphPad Prism software (version 8.0).

### 3. Experimental Results

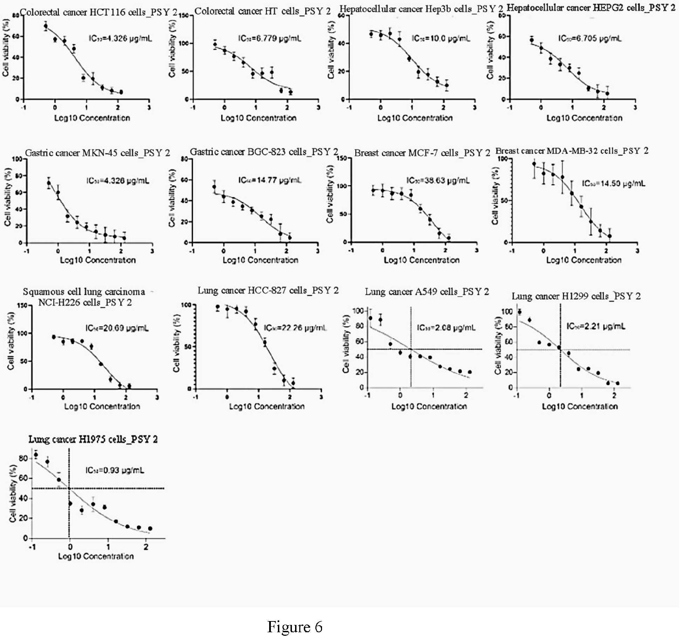

The CCK-8 assay showed the effect of PSY-2 on the proliferation of 13 cancer cell lines. The results are presented in Table 1 and Figure 6. The IC₅₀ values of PSY-2 ranged from 0.8251 to 38.63 µg/mL, demonstrating a favorable dose-response relationship.

**TABLE 1. Inhibitory Effects of Compounds on the Proliferation of Tumor Cell Lines**

| Tumor Type | Cell line | IC₅₀_PSY-2 (µg/mL) | R² |
|---|---|---|---|
| colon cancer | HCT116 | 4.326 | 0.9469 |
| | HT29 | 6.779 | 0.8640 |
| Liver cancer | Hep3B | 10.0 | 0.9189 |
| | HepG2 | 6.705 | 0.8948 |
| Gastric cancer | MKN-45 | 0.8251 | 0.8977 |
| | BGC-823 | 14.77 | 0.8492 |
| Breast cancer | MCF-7 | 38.63 | 0.9176 |
| | MDA-MB-23 | 14.50 | 0.8457 |
| Lung cancer | NCI-H226 | 20.69 | 0.9498 |
| | HCC827 | 22.26 | 0.9545 |
| | H1975 | 0.93 | 0.9024 |
| | A549 | 2.08 | 0.9247 |
| | H1299 | 2.21 | 0.9153 |

The results indicate that compound PSY-2 significantly inhibits the proliferation of tumor cells in colon cancer, liver cancer, gastric cancer, breast cancer, and lung cancer, demonstrating broad-spectrum anti-tumor activity.

### Embodiment 3: Therapeutic Effect of Daoshuilianin A (PSY-2) on Lung Cancer

### 1. Experimental Reagents, Materials, and Instruments:

Cells: LLC mouse lung cancer cells (cultured in F12/DMEM + 10% FBS) obtained from ATCC (ATCC, CRL-1642), and C57BL/6 mice obtained from the Guangdong Provincial Laboratory Animal Center.

Materials and Instruments: Same as in Example 2.

### 2. Experimental Methods:

C57BL/6 mice were randomly divided into two groups (n=5 per group): a model group (LLC) and a drug treatment group (LLC + PSY-2). For the model group Establishment, C57BL/6 mice were subcutaneously injected in the right forelimb with 200 µL of PBS containing LLC cells (3.96X 106 cells/mouse). Mouse body weight was recorded once daily, and tumor volume was measured every two days. For the drug treatment group, C57BL/6 mice was inoculated with LLC cells, and once the tumor volume reached 100 mm³, the mice received daily oral gavage administration of PSY-2 (40 mg/kg) subsequently.

### 3. Experimental Results:

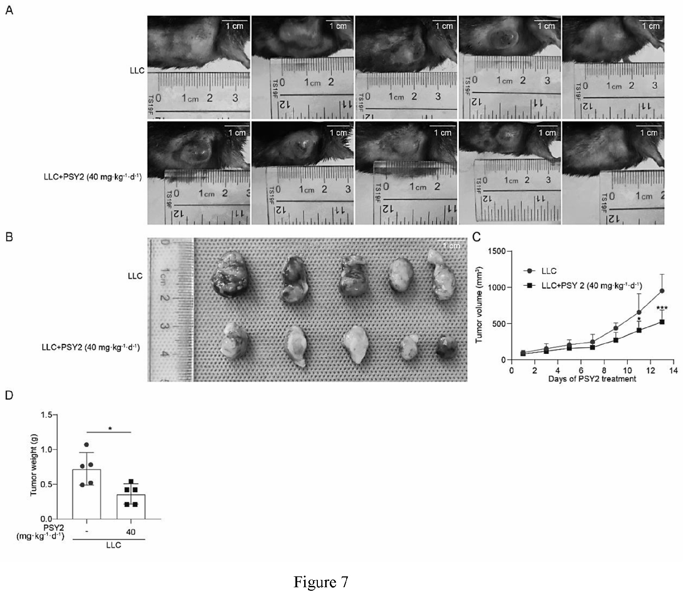

As shown in Figure 7, the treatment group with PSY-2 exhibited a significant reduction in both tumor volume (Figure 7A-7C, P < 0.0001) and tumor weight (Figure 7D, P < 0.05).

### Embodiment 4: Preparation of Daoshuilianin A Tablets

50 g of Daoshuilianin A was mixed with 1900 g of a filter mixture (weight ratio of lactose: starch = 3:2) in a high-shear mixer granulator. The mixture was first blended at low speed (approx. 100 to 200 rpm) for 5 to 10 minutes to ensure uniform dispersion of the active ingredient within the fillers. Then, 50 mL of a 5% (w/v) hydroxypropyl methylcellulose (HPMC) aqueous solution (binder) was added, and mixing continued at medium speed (approx. 300 to 400 rpm) for 10 to 15 minutes to form a soft mass. The mass was granulated through an 80-mesh sieve and dried to control moisture content between 2% and 5%. The dried granules were sized through a 100-mesh sieve. Finally, 40 g of sodium carboxymethyl starch (disintegrant) and 10 g of magnesium stearate (lubricant) were added and mixed thoroughly. The final mixture was loaded into a tablet press hopper and compressed into 10,000 tablets, each containing 5 mg of Daoshuilianin A.

### Embodiment 5: Preparation of Daoshuilianin A Capsule

50 g of Daoshuilianin A was mixed with 1800 g of a filler mixture (weight ratio of microcrystalline cellulose : lactose = 2:1) in a mixer at 25 rpm for 10 minutes. Then, 50 mL of a 5% (w/v) hydroxypropyl methylcellulose (HPMC) aqueous solution (binder) was added, and mixing continued for 15 minutes to form a soft mass. The mass was granulated through an 80-mesh sieve and dried to control moisture content between 2% and 5%. The dried granules were sized through a 100-mesh sieve. Subsequently, 50 g of crospovidone (disintegrant) and 10 g of magnesium stearate (lubricant) were added to the mixer, and the mixture was blended at 20 rpm for 20 minutes. The final mixture was filled into capsules using a capsule filling machine to produce 10,000 capsules, each containing 5 mg of Daoshuilianin A.

### Embodiment 6: Preparation of Daoshuilianin A Injection

50 g of Daoshuilianin A was placed in a clean vessel, and 5 L of PEG-400 was added. The mixture was stirred until the compound was completely dissolved. The solution was then diluted with Water for Injection (WFI) to a final volume of 50 L. The resulting solution was filtered through a 0.22 µm microporous membrane filter. Under aseptic conditions, the filtrate was aseptically filled and sealed into 5 mL ampoules, yielding a total of 10,000 units. The sealed ampoules were sterilized using moist heat sterilization at 121°C for 20 minutes. The final product consists of 5 mL per ampoule, containing 5 mg of Daoshuilianin A.

The embodiments described above are merely preferred examples of the present invention and are not intended to limit the scope of the present invention. Accordingly, any simple modifications, equivalent changes, or alterations made to the above embodiments based on the technical essence of the present invention, without departing from the technical solutions of the present invention, shall fall within the scope of the technical solutions of the present invention.

## Claims

1. An anti-tumor compound having the chemical structure shown in formula (I):

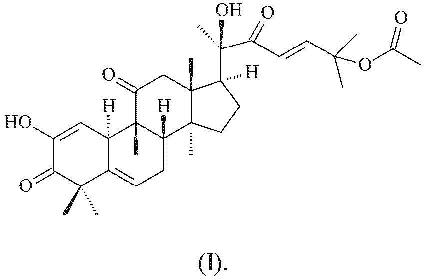

2. A pharmaceutical composition comprising the compound of claim 1.

3. The pharmaceutical composition of claim 2, wherein the pharmaceutical composition further comprises a pharmaceutically acceptable carrier and/or excipient.

4. A method for preparing the compound of claim 1, comprising the following steps:
a. Providing dried rhizomes of *Begonia limprichtii Irmscher,* pulverizing them, extracting with an organic solvent, filtering, and concentrating to obtain a fluid extract;
b. Adding the fluid extract to water, stirring, filtering, washing with water, and drying to obtain a precipitate;
c. Separating the precipitate by silica gel column chromatography, eluting with a 10:0 to 0:10 gradient of petroleum ether-ethyl acetate; and
d. Monitoring the fractions by silica gel column chromatography, combining, and concentrating to obtain the compound.

5. The method of claim 4, wherein the organic solvent is any one selected from methanol, ethanol, ethyl acetate, acetone, chloroform, and petroleum ether.

6. The method of claim 4, wherein the ratio of *Begonia limprichtii Irmscher* to the organic solvent is 1 g : 3 to 5 mL.

7. The method of claim 4, wherein in said step (d), the column chromatography fractions are monitored by thin-layer chromatography or high-performance liquid chromatography.

8. Use of the compound of claim 1 or the pharmaceutical composition of claim 2 in the preparation of an anti-tumor medicament.

9. The use of claim 8, wherein the tumor is any one of lung cancer, breast cancer, liver cancer, colon cancer, rectal cancer, and gastric cancer.

10. The use of claim 8, wherein the compound is formulated into a pharmaceutically acceptable dosage form either as a pure compound or as a plant extract containing the compound.
